# NEUE EUROPÄISCHE PATENTSCHRIFT

(11) **EP 1 102 576 B2**
(45) Veröffentlichungstag und Bekanntmachung des Hinweises auf die Entscheidung über den Einspruch: **14.09.2011**
(45) Hinweis auf die Patenterteilung: 28.06.2006
(21) Anmeldenummer: 99932858.6
(22) Anmeldetag: 09.07.1999
(51) Int. Cl.: A61Q 17/04, A61K 8/35, A61K 8/27, A61K 8/49

(54) **pH-STABILISIERTES ZnO IN SONNENSCHUTZFORMULIERUNGEN**
pH-STABILIZED ZnO IN SUNSCREEN FORMULATIONS
ZnO A pH STABILISE UTILISE DANS DES FORMULATIONS DE PROTECTIONS SOLAIRES

(30) Priorität: 07.08.1998 DE 19835691
(43) Veröffentlichungstag der Anmeldung: 30.05.2001
(73) Patentinhaber: Merck Patent GmbH, 64293 Darmstadt (DE)
(72) Erfinder: KURZ, Thekla, D-64285 Darmstadt (DE); SCHUCHMANN, Heike, D-64380 Ro dorf (DE)
(86) Internationale Anmeldenummer: PCT/EP1999/004830
(87) Internationale Veröffentlichungsnummer: WO 2000/007555

(56) Entgegenhaltungen:
- EP-A- 0 518 772
- EP-A1- 0 557 089
- EP-A1- 0 667 144
- WO-A1-94/28867
- DE-A- 19 754 037
- DE-A1- 4 242 876
- DE-A1- 19 521 951
- US-A- 4 671 956
- US-A- 5 527 519
- '5th Combined Conference of the Australian and New Zealand' 29 März 1998,
- 1998, SPRING CONFERENCE"SUNSCREENS"; SOCIETY OF COSMETIC SCIENTISTS; 12.-23 APRIL 1998,
- Juli 1998, EURO COSMETICS 3,

## Beschreibung

Die vorliegende Erfindung betrifft eine Feststoffmischung gemäß Anspruch 1.

Während vor etwa 30 Jahren Sonnenlicht aufgrund der Vitamin D-Synthese als heilend und unbedenklich angesehen wurde, hat sich in den letzten Jahren die Einstellung in dieser Beziehung nicht nur aus medizinischer Sicht erheblich geändert. Das Gefahrenpotential, welches sowohl natürliche als auch künstliche Bestrahlung mit Sonnenlicht in sich birgt, ist im Bewußtsein in den Vordergrund gerückt. Insbesondere ist auch eine Verhaltensänderung hervorgerufen worden durch das Wissen über den Einfluß von Sonnenlicht auf die Hautalterung und die Entstehung von Hautkrebs.

Bekanntlich reagiert die Haut empfindlich auf Sonnenstrahlen, welche einen gewöhnlichen Sonnenbrand oder ein Erythem, aber auch mehr oder weniger ausgeprägte Verbrennungen hervorrufen können.

Sonnenstrahlen haben aber auch andere negative Wirkungen: sie bewirken, daß die Haut ihre Elastizität verliert und sich Falten bilden und führen somit zu einer frühzeitigen Alterung. Manchmal kann man auch Dermatosen beobachten, und im extremen Fall kommt es zum Auftreten von Hautkrebs.

Es ist auch wünschenswert, Haare gegen photochemische Schäden zu schützen, um Veränderungen von Farbnuancen, ein Entfärben oder Schäden mechanischer Art zu verhindern.

Bekanntlich wird der gefährlichste Teil der Sonnenstrahlen von den ultravioletten Strahlen mit einer Wellenlänge von weniger als 400 nm gebildet. Bekannt ist auch, daß durch das Vorhandensein der Ozonschicht der Erdatmosphäre, die einen Teil der Sonnenstrahlung absorbiert, die untere Grenze der ultravioletten Strahlen, welche die Erdoberfläche erreichen, bei ca. 280 nm liegt.

Daher liegt das Hauptziel im Bereich Sonnenschutz eigentlich darin, einen guten Schutz gegen UVB- und UVA-Strahlung zu gewährleisten.

Innerhalb weniger Jahre haben anorganische Lichtschutzfilter einen festen Platz in der Sonnenkosmetik errungen.

Als geeignete anorganische Lichtschutzflter seien hier Titandioxide, Zinkoxid, Eisenoxide oder auch Ceroxid genannt.

Vermehrt werden seit einigen Jahren auch Zinkoxide als Lichtschutzflter eingesetzt Über einen breiten UV-Bereich von 250 bis 380 nm kann mit solchen anorganischen Lichtschutzfittem ein außerordentlich wirksamer Schutz erzielt werden.

So offenbart EP-A-518 772 kosmetische Zubereitungen enthaltend Zinkoxid und das wasserlösliche Benzylidencampherderivat Dicampherterephthalylidensulfonsäure (Mexoryl^{™} SX).

In US 5,527,519 wird ein pH-stabilisiertes pulverförmiges Zinkoxid durch spezielle fraktionierte Ausfällung hergestellt.

Die anorganischen Filter werden im Sonnenschutz entweder als Pulver oder als Dispersionen in Öl oder Wasser eingesetzt. Mit Dispersionen werden dabei meist höhere Sonnenschutzfaktor-(SPF)-Werte erzielt, da durch eine Naßmahlung (z.B. einer Perlmühle) ein höherer Dispersitätsgrad der Mikropigmente erreicht werden kann.

Znkoxide haben jedoch leider einen Nachteil: sie führen in den Formulierungen zu einer Verschiebung des pH-Wertes zu höheren Werten. Die höheren, ins Alkalische gehenden Werte, können jedoch empfindliche Haut schädigen. Ferner können pH-Wert-Veränderungen auch zum Brechen einer Formulierung führen.

Aufgabe der Erfindung war es daher, ein Zinkoxid mit zusätzlicher pH-Stabilisierung im neutralen Bereich in wässrigen systemen bereitzustellen.

Überraschenderweise wurde nun gefunden, das der Zusatz geringer Mengen eines wasserlöslichen UV-Filters die Stabilität des pH-Wertes erwirken kann, also die Verschiebung ins Alkalische verhindert

Gegenstand der Erfindung ist daher eine Feststoffmischung aus Zinkoxid und einem oder mehreren wasserlöslichen Lichtschutzflter(n), welche dadurch gekennzeichnet ist, das die Dispersion dieser Feststoffmischung in wäßriger Lösung ph-stabil im neutralen Bereich bleibt und als UV-Filter Phenylbenzimidazolsulfonsäure und/oder Benzophenon-4 verwendet werden.

Überraschenderweise reichen schon geringe Menge an Substanz aus, den Shift des pH-Wertes ins Alkalische zu verhindern. Der pH-Wert verbleibt im Bereich von pH 6,6 bis pH 7,2 und ist damit gut hautverträglich.

Als geeignete UV-Filter kommen dabei die / wasserlöslichen UV-Filter Phenylbenzimidazolsulfonsäure (Eusolex® 232) und Benzophenon-4 (Uvinul MS 40) in Frage. Insbesondere bevorzugt wird das Eusolex® 232 verwendet.

Die Herstellung einer kosmetischen Zubereitung enthaltend wenigstens Zinkoxid erfolgt auf sehr einfache Weise, indem man den entsprechenden UV-Filter als Feststoff zumischt. Dabei ist es unerheblich, ob das Pulver der Formulierung während der Herstellung beigegeben wird oder aber schon vorher die erfindungsgemäße Feststoffkombination aus Zinkoxid und UV-Filter nach Anspruch 1 hergestellt und beigegeben wird.

Der Gehalt an wasserlöslichem UV-Filter liegt im Bereich von 0,01 Gew.% bis 8 Gew.-%, vorzugsweise von 0,1 bis 2 Gew.%, bezogen auf das Zinkoxid.

Diese geringe Zugabe von wasserlöslichen UV-Filtern führen also zu einem Zinkoxid, das sich bei der Dispersion in Wasser neutral verhält und auch während der Lagerung keinen pH-Shift verursacht.

Der Gehalt an Zinkoxid als anorganischer UV-Filter wird wie üblich gewählt. In der Regel wird es in einer Menge von 0,5 bis 20 Gew.-%, vor zugsweise 2 -10 Gew.-%, in kosmetische Formulierungen eingearbeitet.

Da die zugesetzten UV-Filter bekannte und erprobte Substanzen sind, gibt es bei der Verwendung in der Kosmetik und bei der Herstellung von kosmetischen Formulierungen und Sonnenschutzmitteln keinerlei Probleme.

Das erfindungsgemäß neutralisierte Zinkoxid kann allein oder natürlich auch in Kombination mit weiteren Lichtschutzfiltern unterschiedlicher Substanzklassen, einzeln oder in Kombination, in der kosmetischen Zubereitung enthalten sein. Lichtschutzfilter anderer Substanzklassen sind beispielsweise organische oder anorganische UVA- und UVB-Filter, IR- oder VIS-Filter. Insbesondere bevorzugt ist die Kombination mit organischen UV-Filtern oder deren Gemischen.

Das erfindungsgemäß neutralisierten Zinkoxid kann in kosmetischen Formulierungen wie Sonnenschutzmitteln, Hautcremes oder Gelen, Haargelen oder kosmetischen Stiften verwendet werden.

Als geeignete organische UV-Filter kommen alle dem Fachmann bekannten UVA- als auch UVB-Filter in Frage. Für beide UV Bereiche gibt es viele aus der Fachliteratur bekannte und bewährte Substanzen, beispielsweise seien hier nur Substanzen wie Benzylidencampherderivate (zB. Eusolex ® 6300), Benzoyl- oder Dibenzoylmethane wie Eusolex® 9020 oder Eusolex® 8020, Benzophenone (Eusolex® 4360), Methoxyzimtsäureester (z.B. Eusolex® 2292), Salicylatderivate (z.B. Eusolex® OS), Octocrylen (Eusolex® OCR), 4-Amino-benzoesäure (PABA), Homosalate (HMS) oder auch Octyl Triazone (Uvinul® T 150) aufgeführt.

Diese organischen UV-Filter werden in der Regel in einer Menge von 0,5 bis 10 Gewichtsprozent, vorzugsweise 1 - 8 %, in kosmetische Formulierungen eingearbeitet.

Als weitere anorganische UV-Filter sind solche aus der Gruppe der Titandioxide oder Eisenoxide denkbar. Diese anorganischen UV-Filter werden in der Regel in einer Menge von 0,5 bis 20 Gewichtsprozent, vorzugsweise 2 - 10 %, in kosmetische Formulierungen eingearbeitet

Gegebenenfalls können die Sonnenschutzmittel auch eine oder mehrere chemische Substanzen mit selbstbräunenden Eigenschaften enthalten.

Als chemische Substanzen mit selbstbräunenden Eigenschaften können alle dem Fachmann bekannten natürlichen und synthetischen Substanzen, welche zur Herstellung von kosmetischen Formulierungen geeignet sind, eingesetzt werden. Solche können sowohl pflanzliche Extrakte als auch synthetische Selbstbräuner, wie zB. Dihydroxyaceton oder α-Ketole, sein.

Weiterhin können die Formulierungen auch zur vorbeugenden Behandlung von Entzündungen und Allergien der Haut sowie auch in bestimmten Fällen zur Verhütung bestimmter Krebsarten verwendet werden.

Die Zubereitung wird als Mittel zum Schutz der menschlichen Epidermis oder der Haare oder auch der sensibilisierten Haare oder als Sonnenschutzmittel verwendet.

Mit "sensibilisierten Haaren" sind Haare gemeint, welche einer Dauerwellenbehandlung, einem Farbe- oder Entfärbeprozeß unterzogen worden sein.

Die kosmetische Zubereitung wird zum Schutz menschlicher Epidermis gegen Sonneneinstrahlung verwendet Dabei liegt sie in verschiedenen, für diesen Typ üblicherweise verwendeten Formen vor. So kann sie insbesondere als Lotion oder Emulsion, wie als Creme oder Milch (O/W, W/O), in Form ölig-alkoholischer, ölig-wäßriger oder wäßrigalkoholischer Gele oder als feste Stifte vorliegen oder als Aerosol konfektioniert sein.

Die Formulierung kann kosmetische Adjuvanzien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfüms, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglycol, Glycerin und Sorbit.

Eine bevorzugt Ausführungsform der Formulierung ist eine Emulsion, welche als Schutzcreme oder -milch vorliegt und außer dem neutralisierten Zinkoxid als UV-Filter - und gegebenenfalls noch weiteren Lichtschutzfiltern - Fettalkohole, Fettsäuren, Fettsäureester, insbesondere Triglyceride von Fettsäuren, Lanolin, natürliche oder synthetische Öle oder Wachse und Emulgatoren in Anwesenheit von Wasser umfaßt.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder ölig-alkoholische Lotionen auf Basis eines Niedrigalkohols, wie Ethanol, oder eines Glycols, wie Propylenglykol, und/oder eines Polyols, wie Glycerin, und Ölen, Wachsen und Fettsäureestern, wie Triglyceriden von Fettsäuren, dar.

Die kosmetische Zubereitung kann auch als alkoholisches Gel vorliegen, welches einen oder mehrere Niedrigalkohole oder - polyole, wie Ethanol, Propylenglycol oder Glycerin, und ein Verdickungsmittel, wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane.

Soll das Mittel natürliche oder sensibilisierte Haare vor Sonneneinstrahlung schützen, so kann es als Shampoo, Lotion, Gel oder Emulsion zum Ausspülen vorliegen, wobei die jeweilige Formulierung vor oder nach dem Schampoonieren, vor oder nach dem Färben oder Entfärben, vor oder nach der Dauerwelle aufgetragen wird; oder das Mittel liegt als Lotion oder Gel zum Frisieren und Behandeln, als Lotion oder Gel zum Bürsten oder Legen einer Wasserwelle, als Haarlack, Dauerwellmittel, Färbe- oder Entfärbemittel der Haare vor. Dieses Mittel kann außer dem neutralisierten Zinkoxid als anorganischen UV-Filter - und gegebenenfalls weiteren Lichtschutzfiltern - verschiedene, in diesem Mitteltyp verwendete Adjuvanzien enthalten, wie grenzflächenaktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Die kosmetischen Zubereitungen können mit Hilfe von Techniken hergestellt werden, die dem Fachmann wohl bekannt sind.

Auch ohne weitere Ausführungen wird davon ausgegangen, daß ein Fachmann die obige Beschreibung in weitesten Umfang nutzen kann. Die bevorzugten Ausführungsformen sind deswegen lediglich als beschreibende, keineswegs als in irgendeine Weise limitierende Offenbarung aufzufassen.

Die vollständige Offenbarung aller vor- und nachstehend aufgeführten Anmeldungen und Veröffentlichungen sind durch Bezugnahme in diese Anmeldung eingeführt.

Die folgenden Beispiele sollen die Erfindung näher erläutern.

### Beispiel 1

In Tabelle 1 werden die Ergebnisse einer Versuchsreihe zur Stabilisierung von ZnO durch Eusolex® 232 in wäßriger Suspension beschrieben.

**Tabelle 1**

| pH-Wert | | | | |
|---|---|---|---|---|
| nach | Herstellung | 1 Woche | 4 Wochen | 9 Wochen |
| 5% ZnO | 7,7 | 7,8 | 7,9 | 8,1 |
| 10 % ZnO | 7,7 | 7,8 | 8,2 | 8,4 |
| 5 % ZnO + 0,1 % 232 | 7,0 | 6,9 | 6,9 | 6,8 |
| 10% ZnO + 0,1 % 232 | 7,1 | 7,0 | 7,0 | 7,1 |
| 5% ZnO + 0,05 % 232 | 7,2 | 7,0 | 7,1 | 7,1 |
| 10% ZnO + 0,05 % 232 | 7,2 | 7,0 | 7,1 | 7,1 |

Es ist deutlich zu erkennen, daß durch die Zugabe von Eusolex® 232 der pH-Wert der wäßrigen Suspension nicht ins Alkalische abweicht, auch nach längerer Lagerung.

### Beispiel 2

Man stellt folgendermaßen eine Sonnenschutzcreme (O/W) mit einem pH-stabilisierten Zinkoxid her:

| | | Gew.-% |
|---|---|---|
| A Eusolex 2292 (Art.-Nr. 1.05382) | (1) | 3,00 |
| Eusolex 9020 (Art.-Nr. 1.05844) | (1) | 2,00 |
| Arlacel 165 | (2) | 10,00 |
| Miglyol 812 Neutralöl (Art.-Nr. 1.06175) | (1) | 20,00 |
| Cetylalkohol (Art.-Nr. 1.00989) | (1) | 2,00 |
| Lanolin Corona | (3) | 2,00 |
| Oxynex 2004 (Art.-Nr. 1.06940) | (1) | 0,05 |
| B ZnO pH-stabil * | (1) | 10,00 |
| Sorbitol F flüssig (Art.-Nr. 1.02993) | (1) | 3,00 |
| Glycerin (Art.-Nr. 1.04093) | (1) | 2,00 |
| Titriplex III (Art.-Nr. 1.08421) | (1) | 0,05 |
| Konservierungsstoffe | (1) | q.s. |
| Wasser, demin. | | ad 100,00 |

| | | |
|---|---|---|
| * ZnO pH-stabil ist eine Pulvermischung und setzt sich zusammen aus 98,0 - 99,9 % ZnO und 0,1 - 2,0 % Eusolex 232 | | |

Als Konservierungsmittel sind
0,050 % Propyl-4-hydroxybenzoat (Art.-Nr. 1.07427) und
0,150 % Methyl-4-hydroxybenzoat (Art.-Nr. 1.06757) enthalten.

### Herstellung:

Die Substanzen der Phase B werden kombiniert und auf 80 °C erwärmt. Phase A wird ebenfalls gemischt und auf 75 °C erwärmt. Dann wird langsam unter vorsichtigem Rühren Phase B zur Phase A gegeben. Man läßt unter Abkühlen und gibt - falsch gewünscht - bei 40 °C Duftstoffe dazu.

### Bezugsquellen:

| | | |
|---|---|---|
| (1) Merck KGaA, Darmstadt | (2) ICI, Essen | (3) Croda, Nettetal |

## Patentansprüche

1. Feststoffmischung aus Zinkoxid und einem oder mehreren wasserlöslichen Lichtschutzfiltern, **dadurch gekennzeichnet, dass** die Dispersion dieser Feststoffmischung in wässriger Lösung pH-stabil im neutralen Bereich bleibt und als UV-Filter Phenylbenzimidazolsulfonsäure und/oder Benzophenon-4 verwendet wird,

2. Feststoffmischung nach Anspruch 1, **dadurch gekennzeichnet, dass** der Gehalt an wasserlöslichem Filter im Bereich von 0,01 bis 8 Gew,-%, vorzugsweise 0,1 bis 2 Gew.%, bezogen auf das Zinkoxid, liegt.

## Claims

1. Mixture of solids comprising zinc oxide and one or more water-soluble light-protection filters, **characterised in that** the dispersion of this mixture of solids remains pH-stable in the neutral range in aqueous solution, and the UV filter used is phenylbenzimidazole-sulfonic acid and/or benzophenone-4.

2. Mixture of solids according to Claim 1, **characterised in that** the content of water-soluble filter is in the range from 0.01 to 8% by weight, preferably 0.1 to 2% by weight, based on the zinc oxide.

## Revendications

1. Mélange de solides comprenant de l'oxyde de zinc et un ou plusieurs filtres de protection vis-à-vis de la lumière hydrosolubles, **caractérisé en ce que** la dispersion de ce mélange de solides reste stable en pH dans la plage neutre en solution aqueuse, et le filtre UV utilisé est l'acide phénylbenzidimidazolesulfonique et/ou benzo-phénone-4.

2. Mélange de solides selon la revendication 1, **caractérisée en ce que** la teneur en filtre hydrosoluble est dans la plage de 0,01 à 8% en poids, de préférence de 0,1 à 2% en poids, sur la base de l'oxyde de zinc.
